# EUROPÄISCHE PATENTSCHRIFT

(11) **EP 3 042 592 B1**
(45) Veröffentlichungstag und Bekanntmachung des Hinweises auf die Patenterteilung: **30.01.2019**
(21) Anmeldenummer: 15200636.7
(22) Anmeldetag: 17.12.2015
(51) Int. Cl.: A47J 31/44

(54) **VERFAHREN ZUM ERZEUGEN VON MILCHSCHAUM UND VERWENDUNG EINES HEISSGETRÄNKEAUTOMATEN ZUM HERSTELLEN EINES KAFFEE-MILCH-MISCHGETRÄNKES**
METHOD FOR PRODUCING MILK FROTH AND USE OF A HOT BEVERAGE MACHINE FOR PRODUCING A MIXED COFFEE-MILK BEVERAGE
PROCÉDÉ DE PRODUCTION DE MOUSSE DE LAIT ET UTILISATION D'UNE MACHINE DE BOISSON CHAUDE POUR FABRICATION D'UN BOISSON MÉLANGÉE À BASE DE LAIT ET DE CAFÉ

(30) Priorität: 18.12.2014 DE 102014119062
(43) Veröffentlichungstag der Anmeldung: 13.07.2016
(73) Patentinhaber: Melitta Professional Coffee Solutions GmbH & Co. KG, 32429 Minden (DE)
(72) Erfinder: BUCHHOLZ, Bernd, 32369 Rahden (DE); DIESTER, Thomas, 31675 Bückeburg (DE)
(74) Vertreter: Specht, Peter

(56) Entgegenhaltungen:
- EP-A1- 2 294 952
- EP-A1- 2 583 596
- DE-A1-102013 104 339

## Beschreibung

Die Erfindung betrifft ein Verfahren, mit dem Milch-Luft-Emulsionen, insbesondere Milchschäume, mit einer Vorrichtung zum Erzeugen eines Milchschaums hergestellt werden und eine Verwendung eines Heissgetränkeautomaten zum Herstellen eines Kaffee-Milch-Mischgetränkes.

Die Erzeugung bzw. "Herstellung" von Milchschaum, d.h. eines jedenfalls vorübergehend relativ stabilen Gemisches aus Milch und Luft mit ggf. einer entsprechenden Erwärmung, ist aus dem Stand der Technik in verschiedensten Ausführungsformen bekannt und kommt beispielsweise in Kaffeevollautomaten zur Anwendung. Die EP 2 298 142 B1 offenbart eine Vorrichtung zum Aufschäumen von Milch, die zur Steuerung der zugeführten Luftmenge ein intermittierendes Luftventil umfasst. Das Luftventil ist ein Magnetventil, das zwischen zwei Zuständen umschaltbar ist. Mit dem Luftventil wird der mittlere Luftdurchlass durch wiederholtes Umschalten zwischen den beiden Zuständen des Luftventils gesteuert. Die Einstellung der Luftzufuhr über entsprechende Bauteile wie beispielsweise Nadel-Magnetventile (z. B. Injektionsventile) ist allerdings aufwendig und führt auch oftmals nicht zu den gewünschten Ergebnissen.

Aus der US 6 192 785 ist es bekannt, kalten Milchschaum über einen ersten Leitungsabschnitt mit einer ersten Drosselanordnung zu einem Auslass zu fördern und heißen Milchschaum über einen dazu parallelen zweiten Leitungsabschnitt über eine zweite Drosselanordnung und einen Wärmeüberträger zum Auslass zu fördern.

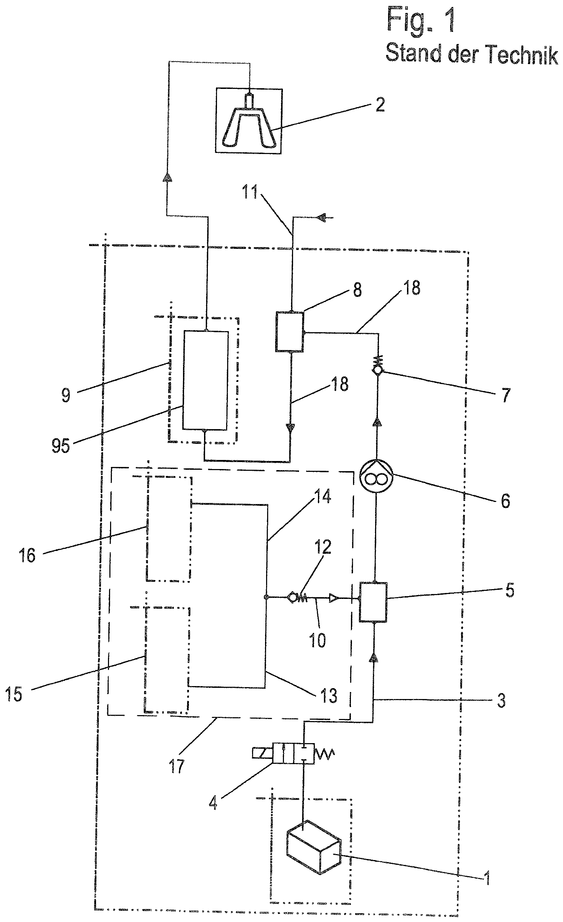

Es ist es auch bekannt, die Luft der Milch durch verschiedene Zuleitungen ansaugend zuzuführen, in denen Drosseln verschiedenen Öffnungsquerschnitts vorgesehen sind. Hier wird nicht die Luftmenge, die in die Milch gepumpt wird variiert sondern es wird über verschiedene Querschnitte verschieden viel Milch in die Luft gesaugt. Die DE 10 2013 104 339 A1 offenbart eine solche vorteilhafte Vorrichtung und ein zugehöriges Verfahren zur Herstellung von Milchschaum. Die dort beschriebene Vorrichtung zur Herstellung des Milchschaums zeigt die Fig. 1. Bei dem Verfahren zur Herstellung von Milchschaum wird zum Fördern von Milch eine zur Veränderung der Förder- und Pumpmenge an Milch einstellbare Milchpumpe verwendet. Die Milchpumpe ist hinsichtlich ihrer Drehzahl veränderbar. Dadurch ist nicht nur die Fördermenge der Milch in einfacher Weise einstellbar. Sondern zudem können durch die Variation der Drehzahl die Eigenschaften des hergestellten Milchschaums, insbesondere seine Konsistenz, durch Ansaugen verschiedener Luftmengen variiert werden. Um mit der Vorrichtung sowohl warmen als auch kalten Milchschaum herstellen zu können, weist sie zwei Zweigleitungen für die Luftzufuhr auf, die jeweils an eine Luftquelle angeschlossen sind. In den Zweigleitungen sind Absperrventile und/oder Drosseln verschiedenen Öffnungsquerschnitts angeordnet, um die der Milch zugeführte Luftmenge verändern zu können. Mit Hilfe einer Dampfquelle kann ein erzeugter kalter Milchschaum erhitzt werden.

Die Erfindung hat die Aufgabe, ein Verfahren zur Herstellung von Milchschaum zu schaffen, mit denen auf einfache Weise mehr verschiedene Milchschäume erzeugt werden können als bei dem gattungsgemäßen Stand der Technik der DE 10 2013 104 339.

Die Erfindung löst diese Aufgabe durch den Gegenstand des unabhängigen Patentanspruchs 1. Die Erfindung schafft zudem den vorteilhaften Gegenstand des Anspruchs 11. Vorteilhafte Ausführungsformen sind in den abhängigen Ansprüchen genannt.

Nach Anspruch 1 wird zunächst folgendes Verfahren geschaffen:
Verfahren, mit dem Milch-Luft-Emulsionen, insbesondere Milchschäume, mit einer Vorrichtung zum Erzeugen eines Milchschaums hergestellt werden, wobei die Vorrichtung zumindest aufweist: a) eine von Milch durchflossenen Milchleitung (3), b) wenigstens eine Einrichtung zur Erzeugung einer Milch-Luft-Emulsion als kalter Milchschaum - KMS - mit einer ersten Temperatur T1, c) eine Dampfquelle (22) zur Injektion von Heißdampf in eine Milch-Luft-Emulsion erster Temperatur T1 auf eine heiße Temperatur T3 > T1 zum Erzeugen eines heißen Milchschaums - HMS - mit der Temperatur T3, so dass mittels Dampfinjektion der heiße Milchschaum - HMS - auf die Temperatur T3 insbesondere zwischen 55° C und 80°C erhitzbar ist, und d) einen Wärmeüberträger (20) zum Erwärmen einer Milch-Luft-Emulsion erster Temperatur T1 auf eine warme Temperatur T2 zum Erzeugen eines warmen Milchschaums - WMS - mit der Temperatur T2, wobei gilt: T1 < T2 < T3; wobei mit dem Wärmeüberträger (20) der Milchschaum auf die Temperatur T2 auf eine denaturierend wirkende Temperatur, insbesondere auf zwischen 40° C und 54°C erwärmbar ist, mit zumindest folgenden Schritten:
A) Fördern von Milch in einer Milchleitung (3) mittels einer Milchpumpe (6), und gleichzeitig Ansaugen von Luft durch eine Luftleitung (10, 13, 14),
B) Mischen von Milch und Luft - vorzugsweise in einer ersten Mischkammer (5) - zum Erzeugen einer Luft-Milch-Emulsion als kalter Milchschaum - KMS - mit einer Temperatur T1,
C) Zusetzen von Dampf zur Luft-Milch-Emulsion zum Erhitzen der Luft-Milch-Emulsion auf eine heiße Temperatur T3, um einen heißen Milchschaum - HMS -mit der heißen Temperatur T3 > T1 erzeugt werden soll, mittels Dampfinjektion der heiße Milchschaum - HMS - auf die heiße Temperatur T3 - insbesondere zwischen 55° C und 80°C - erhitzt wird;
D) wobei vor oder nach Schritt c) ein Erwärmen der Luft-Milch-Emulsion mit der Temperatur T1 mit einem Wärmeüberträger auf eine warme Temperatur T2 > T1 erfolgt, um ferner einen warmen Milchschaum - WMS - mit der warmen Temperatur T2 zu erzeugen, wobei gilt: T1 < T2 < T3, wobei mit dem Wärmeüberträger der warme Milchschaum - WMS - auf die warme Temperatur T2 insbesondere zwischen 40° C und 54°C erwärmt wird.

Mit der erfindungsgemäßen Vorrichtung und dem erfindungsgemäßen Verfahren können auf einfache Weise kalter Milchschaum (KMS), warmer Milchschaum (WMS) und heißer Milchschaum (HMS) in beliebiger Reihenfolge ausgegeben werden. Wird die Luftzufuhr gestoppt, können zudem kalte Milch und heiße Milch ausgegeben werden.

Der warme Milchschaum (WMS) ist eine spezielle Milchschaumart, welche durch die definierten Anforderungen an Konsistenz, Stabilität, Thermorezeption und Optik, eine indirekte Erwärmung des kalten Milchschaums voraussetzt. (Eigenschaften die nicht durch das direkte Einbringen eines weiteren Mediums z.B. direkte Dampfinjektion verändert werden). Vorzugsweise beträgt die Temperatur des kalten Milchschaums (KMS) < 30° C, des warmen Milchschaums (WMS) 40° bis 54° C und des heißen Milchschaums (HMS) 55° bis 80° C. Der heiße Milchschaum (HMS) und die heiße Milch können mittels direkter Dampfinjektion sehr schnell sehr hoch erhitzt werden. (Hohe Ausgabeleistung bei hoher Temperatur (55-80 °C)).

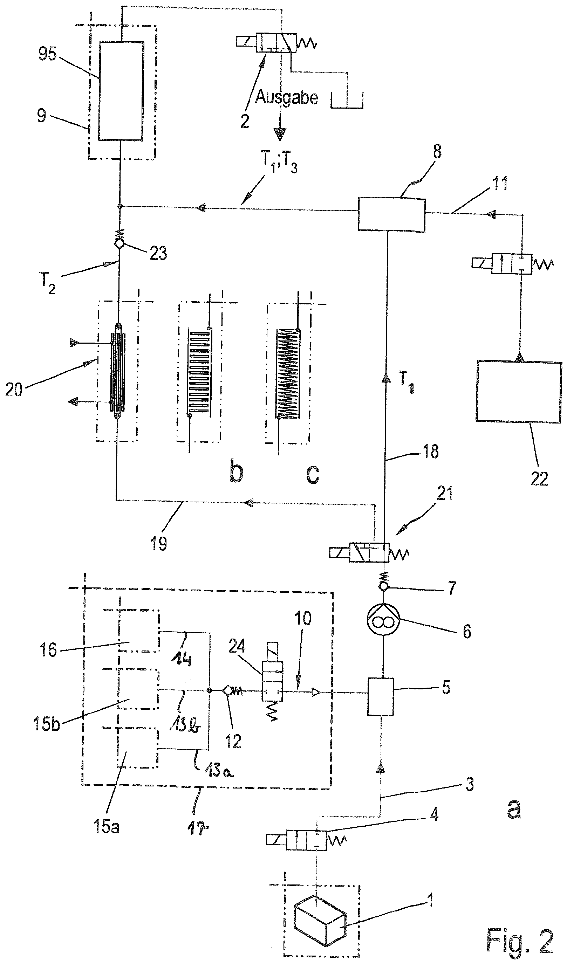

Der kalte Milchschaum (KMS) kann im System der Fig. 1 in seinen Eigenschaften vorzugsweise über die Pumpendrehzahl verändert werden (Fig.1, ist auch Fig.2 realisierbar).

Der warme Milchschaum (WMS) kann auf Grund seiner Konsistenz und Stabilität sehr gut zur optischen Optimierung von Getränken genutzt werden, ohne das Getränk wesentlich abzukühlen oder die Thermorezeption negativ zu beeinflussen.

Dies bedeutet, dass der warme Milchschaum (WMS) vorzugsweise auf ein Getränk, insbesondere einen Kaffee quasi als optische Abdeckung aufgebracht wird. Davor oder danach wird in das Getränk heiße Milch oder danach heißer Milchschaum (HMS) eingegeben, der sich mit dem Getränk mischt, so dass es nicht durch die Schaumzugabe zu stark abkühlt. Diese überraschenden Vorteile des Bereitstellens sowohl warmen als auch heißen Milchschaums (WMS; KMS) oder heißer Milch wurden bisher nicht erkannt.

Dabei ist es vorteilhaft und zweckmäßig, wenn die Vorrichtung wenigstens eine Milchpumpe, insbesondere eine hinsichtlich ihrer Drehzahl einstellbare Milchpumpe, zur Förderung der Milch aufweist.

Es ist ferner vorteilhaft und einfach, wenn die Einrichtung zur Erzeugung des Milchschaums mit der ersten kalten Temperatur T1 wenigstens eine Mischkammer zum Mischen von Milch und Luft aufweist, zum Erzeugen einer Luft-Milch-Emulsion als der kalte Milchschaum (KMS) T1 mit der Temperatur T1.

Um die Konstruktion einfach zu gestalten, ist es ferner vorteilhaft, wenn die Dampfquelle in einen ersten Leitungsabschnitt der Milchleitung mündet und wenn der Wärmeüberträger, welcher die Luft-/Milchemulsion auf T2 erwärmt, in einem dazu parallel geschalteten Leitungsabschnitt angeordnet ist. Dabei ist es zweckmäßig, wenn ein Umschaltventil zum Umschalten zwischen den beiden Leitungsabschnitten vorgesehen ist.

Zur Erzeugung eines Schaums guter Qualität hat es sich ferner als vorteilhaft erwiesen, wenn die beiden parallel geschalteten Leitungsabschnitte hinter der Milchpumpe angeordnet sind.

Dabei ist es zum Verfeinern der Schaumqualität in Hinsicht auf die drei Milchschäume KMS, WMS und HMS der Temperaturen T1, T2 und T3 vorteilhaft, wenn zwischen den beiden Leitungsabschnitten und einem Auslass für die Milch-Luft-Emulsion ein Homogenisator zum Verfeinern des Schaums angeordnet ist.

Es ist zudem zweckmäßig, wenn die Vorrichtung eine Luftleitung zum Zuführen von Luft für kalten Milchschaum KMS, eine Luftleitung zum Zuführen von Luft für warmen Milchschaum WMS und eine Luftleitung zum Zuführen von Luft für heißen Milchschaum HMS aufweist. Dabei kann in zumindest einer oder jeder der Luftleitungen eine Drossel, insbesondere eine einstellbare Drossel angeordnet sein.

Besonders vorteilhaft ist ferner, wenn die ggf. verwendete Drossel hinsichtlich der Größe ihres Öffnungsquerschnitts stufenlos und ggf. automatisch einstellbar ausgebildet ist. Die stufenlose Einstellung des Öffnungsquerschnitts der Drossel ermöglicht die Einstellung der der Milch zugeführten Luftmenge in Abhängigkeit von der gewünschten Milch-Luft-Emulsion (z.B. kalt, warm, heiß). Dadurch können mit nur einer einstellbaren Drossel Milch-Luft-Emulsionen, insbesondere Milchschäume, mit unterschiedlichen Eigenschaften hergestellt werden.

Es ist bevorzugt, dass der Milch jedenfalls zur Herstellung der heißen Milch-Luft-Emulsion weniger Luft zugeführt wird als zur Herstellung der kalten Milch-Luft-Emulsion. Der Öffnungsquerschnitt der Drossel ist daher während der Herstellung der heißen Milch-Luft-Emulsion bevorzugt kleiner, als bei der Herstellung der kalten Milch-Luft-Emulsion. Weiterhin kann die Konsistenz der Milch-Luft-Emulsion weitgehend unabhängig von der Temperatur durch Änderung des Öffnungsquerschnitts variiert werden.

Dabei ist es weiterhin bevorzugt, dass die Förder- und Pumpmenge der Milchpumpe gesteuert wird. Dadurch ist die Konsistenz des Milchschaums veränderbar.

Es ist bevorzugt, dass die Vorrichtung eine Gerätesteuerung aufweist und dass diese dazu eingerichtet ist, den Öffnungsquerschnitt jeweils vor der Herstellung einer Milch-Luft-Emulsion einmalig einzustellen. Ein Produkt kann dabei mehrere verschiedene Bestandteile von Milch-Luft-Emulsionen oder Milch haben.

In einer bevorzugten Ausführungsform ist die Gerätesteuerung zudem dazu ausgelegt, die Förder- und Pumpmenge der Milchpumpe vor der Herstellung einer Milch-Luft-Emulsion einer definierten Eigenschaft einzustellen. Während der Herstellung mit bekanntem Öffnungsquerschnitt der einstellbaren Drossel und einer bekannten für die Herstellung vorgegebenen Fördermenge der Milchpumpe sind die Eigenschaften wie z. B. Konsistenz und Temperatur der hergestellten Milch-Luft-Emulsion reproduzierbar, und daher kontrollierbar.

Mit der Geräteansteuerung werden zudem die Dampfquelle und der Wärmeüberträger angesteuert und weitere ansteuerbare Komponenten wie Umschaltventile und dgl.

Vorteilhaft und bevorzugt ist ein Schließen der Luftleitung dadurch realisierbar, dass mit der einstellbaren Drossel der Öffnungsquerschnitt schließbar ist und geschlossen wird. Es ist aber auch ein Versperren mittels ansteuerbaren Ventilen denkbar.

Die Vorrichtung und das Verfahren ermöglichen zusammenfassend zumindest die Herstellung von Milch-Luft-Emulsionen drei verschiedener Temperaturen mit einer weitgehenden Einstellung definierter Eigenschaften wie z. B. Temperatur und Konsistenz.

Vorteilhaft eingesetzt wird dabei jeweils ein Heißgetränkeautomat, insbesondere ein Kaffeevollautomat, mit einer Vorrichtung zum Erzeugen einer Milch-Luft-Emulsion, insbesondere Milchschaums. Dabei ist es bevorzugt, dass die Milch-Luft-Emulsionen allein ausgegeben werden, oder gemeinsam mit einem Heißgetränk.

Die Erfindung schafft zudem eine Verwendung eines Heissgetränkeautomaten zum Herstellen eines Kaffee-Milch-Mischgetränkes nach Anspruch 11. Genutzt werden dabei ein Heißgetränkeautomat mit einer Vorrichtung zum Erzeugen von Milchschaum und mit einer Kaffee-Zubereitungseinrichtung. Dabei wird auf und in einen (mit der Kaffee-Zubereitungseinrichtung bereitgestellten) Kaffee wenigstens ein warmer Milchschaum (WMS) und ein heißer Milchschaum (HMS) mit verschiedener Temperatur T2 und T3 ein- und zugegeben werden. Vorzugsweise wird der warme Milchschaum (WMS) T2 überwiegend auf den Kaffee gegeben und der heiße Milchschaum (HMS) T3 vorzugsweise durch den warmen Milchschaum (WMS) T2 überwiegend in den Kaffee. Der heiße Milchschaum (HMS) vermengt sich überwiegend mit dem Kaffee, der warme Milchschaum (WMS) überwiegend nicht. Bereitgestellt wird schließlich das Kaffee-Milch-Mischgetränk aus Kaffee und den zwei verschieden temperierten Milchschäumen.

Mit dem Heißgetränkeautomaten, insbesondere einem Kaffeevollautomaten, und dem bzw. den erfindungsgemäßen Verfahren sind vorteilhafte Getränkespezialitäten realisierbar. Besonders vorteilhaft kann ein hervorragender "Cappuccino" erzeugt werden. Dazu weist der Heißgetränkeautomat eine Einrichtung zum Brühen von Kaffee und zum Ausgeben des Kaffees auf. Zuerst wird der Kaffee, insbesondere ein Espresso, von der Maschine in die Tasse gegeben, wo sich die Crema ausbilden kann. Anschließend wird der warme Milchschaum (WMS) genutzt, um diese an den Rand der Tasse zu drängen und somit ein ansprechendes Erscheinungsbild zu generieren. Anschließend wird mit heißem Milchschaum (HMS), welcher die warme Milchschaumhaube durchdringt und in den Kaffee fließt, der Espresso gemischt, um einen "cremigen" "Milchschaum-Kaffee" Geschmack zu erzeugen.

Nachfolgend wird die Erfindung unter Bezug auf die Zeichnung anhand eines Ausführungsbeispiels näher beschreiben. Es zeigt:
- Fig. 1: eine schematische Darstellung des Aufbaus einer Vorrichtung zum Herstellen von Milch-Luft-Emulsionen bzw. - Gemischen, insbesondere von Milchschaum, nach dem Stand der Technik;
- Fig. 2: in a) eine schematische Darstellung des Aufbaus einer ersten erfindungsgemäßen Vorrichtung zum Herstellen von Milch-Luft-Emulsionen, insbesondere von Milchschaum und in b) und c) Varianten von Wärmeüberträger für die Vorrichtung aus a)

Fig. 1 zeigt die in der DE 10 2013 104 339 A1 offenbarte Vorrichtung zur Herstellung von auf Milch basierenden Produkten.

Im Folgenden werden die Begriffe Milch-Luft-Gemisch und Milch-Luft-Emulsion und Milchschaum synonym verwendet.

### Die Vorrichtung der Fig. 1 weist folgenden Aufbau auf:

Eine Milchleitung 3 verbindet einen Milchbehälter 1 und eine Auslassvorrichtung 2 miteinander. Die Auslassvorrichtung 2 ist als Auslauf ausgebildet. Im Folgenden werden die Begriffe Auslassvorrichtung 2 und Auslauf synonym verwendet. Dabei sind in die Milchleitung 3 zwischen dem Milchbehälter 1 und dem Auslauf 2 in dieser Reihenfolge hier folgende Komponenten geschaltet:
ein Absperrventil 4, eine erste Mischkammer 5, eine Milchpumpe 6, ein erstes Rückschlagventil 7, eine zweite Mischkammer 8 und eine Nachbehandlungsvorrichtung 9.

Die Vorrichtung weist zudem ein Luftzuführsystem 17 auf, mit einer Luftleitung 10, die in die erste Mischkammer 5 mündet. In die Luftleitung 10 ist ein zweites Rückschlagventil 12 geschaltet. Sie verzweigt sich vor dem zweiten Rückschlagventil 12 in beispielsweise zwei Zweigleitungen 13, 14. Beide Zweigleitungen 13, 14 sind mit einer Luftquelle 15 oder 16 (z.B. einer Luftpumpe oder einem Druckbehälter oder einem nicht unter Druck stehenden Behälter oder durch eine Öffnung bzw. ein Leitungsende (saugend) mit der Umgebungsluft innerhalb oder außerhalb einer umgebenden Maschine) verbunden.

Vorzugsweise sind in die Zweigleitung 10 Vorrichtungen wie ein Absperrventil (nicht gezeigt), eine oder mehrere herkömmliche Drosseln (nicht gezeigt) und/oder dgl. geschaltet, um die Luftzufuhr zu starten und unterbrechen zu können. Eine herkömmliche Drossel bietet den Vorteil, dass stets eine genau definierte Luftmenge zugegeben wird. Solche Drosseln sind dem Fachmann an sich bekannt und sind daher in dieser Figur nicht dargestellt. Die Anordnung der beiden Zweigleitungen 13, 14 ermöglicht durch getrennte oder gemeinsame Nutzung der beiden (oder in Fig. 2 mehr) Zweigleitungen 13, 14, und/oder durch die Verwendung von herkömmlichen Drosseln mit verschiedenem Öffnungsquerschnitt eine Veränderung der zugeführten Luftmenge.

In die zweite Mischkammer 8 mündet ferner eine Dampfzuleitung 11, durch die heißer Wasserdampf aus einer Wasserdampfquelle (hier nicht dargestellt) in die Milchleitung 3 geleitet werden kann.

### Die Funktion dieser Vorrichtung ist wie folgt:

Mittels der in die Milchleitung 3 geschalteten Milchpumpe 6 wird Milch aus dem Milchbehälter 1 zum Auslauf 2 gefördert. Dieses Fördern erfolgt vorzugsweise während der Dauer der Milchschaumherstellung kontinuierlich.

Dabei wird das Absperrventil 4 zunächst von der dargestellten Sperrstellung in seine Durchlassstellung geschaltet.

Die Milch wird sodann aus dem Milchbehälter 1 in und durch die erste Mischkammer 5 gefördert. In der ersten Mischkammer 5 - in einfacher Form beispielsweise nach Art eines T-Stückes mit einem im Durchmesser vergrößerten Mischkammerbereich ausgestaltet (hier nicht dargestellt) - wird in die durch die Milchleitung 3 strömende Milch Luft geleitet, derart, dass sich die Milch mit der zuströmenden Luft zu einem schaumartigen Milch-Luft-Gemisch vermischt. Das Milch-Luft-Gemisch ist emulsionsartig ausgebildet, so dass in der Milch Luftbläschen eingeschlossen sind.

Der durch die erste Mischkammer 5 strömenden Milch wird während der Milchschaumerzeugung kontinuierlich eine konstante Menge Luft zugesetzt, um ein Milch-Luft-Gemisch möglichst gleichbleibender Eigenschaft zu erzeugen. Das in der ersten Mischkammer erzeugte Milch-Luft-Gemisch wird durch die Milchpumpe 6 gefördert, in welcher sie verdichtet wird. Dabei wird eine hinsichtlich der Fördermenge einstellbare Milchpumpe 6 verwendet.

Das aus der Milchpumpe 6 aus- und weiterströmende, verdichtete Milch-Luft-Gemisch wird durch einen Leitungsabschnitt, eine Dekompressionsstrecke 18, in welche die zweite Mischkammer 8 zwischengeschaltet ist, gefördert. In der zweiten Mischkammer 8, die beispielsweise nach Art eines T-Stückes ohne Drosselfunktion ausgebildet ist, wird dem Milch-Luft-Gemisch bzw. der Milch-Luft-Emulsion optional aus einer Dampfquelle 22 in die Leitung 11 zuströmender heißer Wasserdampf zugesetzt, um die Temperatur des verdichteten Mich-Luft-Gemisches zu erhöhen und einen auf eine Temperatur T3 erwärmten "heißen" Milchschaum HMS zu erzeugen. Dabei ist die Menge des zuströmenden Wasserdampfes vorzugsweise veränderbar, um den Grad der Erwärmung des Milch-Luft-Gemisches anpassen zu können. Wird kein Wasserdampf zugesetzt, wird ein "kalter" Milchschaum KMS mit einer Temperatur T1 < T2 erzeugt. An die zweite Mischkammer 8 schließt sich ein weiterer Abschnitt der Dekompressionsstrecke 18 an, der in die Nachbehandlungsvorrichtung 9 mündet.

Das aus der zweiten Mischkammer 8 und dem Leitungsabschnitt "Dekompressionsstrecke 18" ausströmende kalte oder heiße Milch-Luft-Gemisch mit der Temperatur T1 oder T2 wird zunächst in der Nachbehandlungsvorrichtung 9 nachbehandelt, um die Schaumqualität gezielt zu verbessern.

Die Nachbehandlungsvorrichtung 9 weist einen Homogenisator 95 auf, der dazu vorgesehen ist, den Milchschaum zu homogenisieren. Homogenisieren bedeutet in diesem Zusammenhang, dass die Luftbläschen verkleinert werden. Hierzu weist der Homogenisator 95 beispielsweise Prallkörper auf, welche die Luftbläschen verkleinern und zerteilen, wobei der Homogenisator nicht als hydraulische Drossel(-anordnung) im Sinne des Standes der Technik wirkt.

Die Art und Intensität der Nachbehandlung ist dabei besonders von der Förderleistung bzw. der Drehzahl der Milchpumpe 6 abhängig, also von dem Druck, mit welchem die Milchpumpe 6 den Milchschaum in die Nachbehandlungsvorrichtung 9 hineinpumpt.

Aus der Nachbehandlungsvorrichtung 9 wird der homogenisierte heiße oder kalte Milchschaum 40 durch den Auslauf 2 in ein Gefäß (nicht gezeigt), beispielsweise in eine Tasse, ausgegeben.

Anzumerken ist noch, dass nach den Ausführungsbeispielen der Fig. 1 und 2 jeweils eine Geräte-Steuerung(svorrichtung) (nicht dargestellt) vorgesehen ist. Vorzugsweise und vorteilhaft sind verschiedene Kombinationen von Drosselquerschnittseinstellungen für die Luftzufuhr in einer Steuerung hinterlegt und für die Einstellung von Produktbestandteilen anwählbar. Es ist ferner vorteilhaft und bevorzugt, wenn verschiedene Kombinationen von Drosselquerschnittseinstellungen und/oder Pumpendrehzahl(en) in der Steuerung hinterlegt und für die Einstellung von Produktbestandteilen anwählbar sind.

Die in Fig. 2 dargestellte bevorzugte Variante einer erfindungsgemäßen Vorrichtung entspricht weitgehend der Vorrichtung der Fig.1, so dass auf deren Aufbau verwiesen werden kann. Unterschiede werden nachfolgend erörtert.

Die Vorrichtung der Fig.2 weist auch wie die Vorrichtung der Fig. 1 ein Luftzuführsystem 17 auf, mit einer Luftleitung 10, die in die erste Mischkammer 5 mündet. In die Luftleitung 10 ist wiederum ein zweites Rückschlagventil 12 geschaltet. Sie verzweigt sich vorzugsweise vor dem zweiten Rückschlagventil 12 in beispielsweise zwei oder aber sogar drei Zweigleitungen 13a, 13b, 14. Die Zweigleitungen 13a, 13b, 14 sind vorzugsweise wiederum mit einer Luftquelle 15a, 15b, 16 (einer Luftpumpe oder einem Druckbehälter oder einem nicht unter Druck stehenden Behälter oder dgl. oder die durch eine Öffnung ansaugbare Umgebungsluft innerhalb oder außerhalb einer die Vorrichtung umgebenden Maschine) verbunden.

Die Vorrichtung der Fig. 2 unterscheidet sich von der Vorrichtung der Fig. 1 ferner insbesondere durch einen Leitungsabschnitt 19, der parallel zum Leitungsabschnitt "Dekompressionsstrecke 18" geschaltet ist. Dieser zweite Leitungsabschnitt dient wiederum als eine Art Dekompressionsstrecke zur Erzeugung eines Milchschaums warmer, mittlerer, Temperatur T2, wobei folgende Beziehung gilt: T1 < T2 < T3.

In die zweite Dekompressionsstrecke 19 (Fig. 2) ist ein Wärmeüberträger 20 geschaltet. Dieser Wärmeüberträger 20 ist beispielsweise ein Wärmetauscher, der wenigstens einen oder zwei oder mehrere parallele oder in Reihe vorgesehene Leitungsabschnitte aufweist, der/die von einem erwärmten Medium umspült ist/sind. Derart kann das durch die zweite Dekompressionstrecke - Leitungsabschnitt 19 - strömende Milch-Luftgemisch auf eine mittlere Temperatur T2 erwärmt werden.

Mit Hilfe eines Umschaltventils 21, hier eines 2-Wegeventils, kann hinter der Milchpumpe 6 und vorzugsweise hinter dem Rückschlagventil 7 zwischen der ersten Kompressionsstrecke 18 und der zweiten Dekompressionsstrecke 19 bzw. den beiden parallelen Leitungsabschnitten mit und ohne den Wärmeüberträger 20 umgeschaltet werden (Fig. 2). Der Leitungsabschnitt 19 führt in den Homogenisator 95 oder mündet vorzugsweise vor dem Homogenisator wieder in den Leitungsabschnitt 18. In dem Leitungsabschnitt 19 kann hinter dem Wärmeüberträger ein weiteres Rückschlagventil 23 geschaltet sein.

Vorzugsweise ist der Wärmeüberträger 20 dazu ausgelegt, durchfließende Milch-Luft-Emulsion bzw. den kalten Milchschaum der Temperatur T1 auf eine Temperatur T2 zu erwärmen, die oberhalb der Temperatur T1 liegt, welche der kalte Milchschaum KMS aufweist und unterhalb der Temperatur T3, welche der heiße Milchschaum HMS aufweist. Derart wird ein "warmer" Milchschaum WMS erzeugt.

Durch den Leitungsabschnitt 19 fließt damit ausschließlich warmer Milchschaum WMS und den Leitungsabschnitt 18 fließt ausschließlich kalter oder heißer Milchschaum KMS, HMS oder heiße oder kalte Milch ohne Schaum.

Der Wärmeüberträger 20 (Fig. 2) ist vorzugsweise ein dampfbetriebener oder wahlweise/vorzugsweise fluidbetriebener indirekter Wärmeüberträger der, wenn aktiviert, die Milch-Luft-Emulsion indirekt auf eine Temperatur T2 erwärmt. Die Temperatur T2 liegt vorzugsweise zwischen 40 °C und 544 °C. Sie ist jedenfalls so hoch, dass sie denaturierend wirkt.

Der Wärmeüberträger 20 kann in verschiedenster Weise, so als Mantelrohr-Wärmeüberträger (Fig. 2a), als Schicht-Wärmeüberträger (Fig. 2b) oder als Spiralrohr- Wärmeüberträger (Fig. 2c) ausgebildet sein. Die Wärmeübertragung findet nach der Herstellung des kalten Milchschaums in der Pumpe 6 und vor der Ausgabe statt. Vorteilhaft ist, wenn der warme Schaum WMS mit der Temperatur T2 wie der kalte Milchschaum KMS mit der Temperatur T1 und der heiße Schaum HMS mit der Temperatur T3 einer optionalen Nachbehandlung im Homogenisator 95 unterzogen werden.

Der Wärmeüberträger 20 kann nach einer vorteilhaften Variante derart ausgelegt sein, dass in seinem Bereich der Volumenstrom des Milchschaums in der Einheit reduziert wird, um somit eine vorteilhafte Baugröße im Verhältnis zur Wärmeübertragung zu ermöglichen.

Die eingebrachte Wärmeenergie wird vorzugsweise über eine oder mehrere Kontaktheizflächen an den Milchschaum übertragen, in dieser Funktion ist zur Vergrößerung der Kontaktfläche und somit Wärmeübertragung auch ein zusätzlicher innerer oder äußerer Erwärmungskanal denkbar, sowie die Abfrage der Temperatur, im inneren des Elements, mit einem Fühler. Vorzugsweise dient Wasserdampf als Wärmeträger.

Die Konsistenz des noch kalten Milchschaums KMS mit der Temperatur T1, der aus der Pumpe 6 strömt, wird durch Einstellen der Fördermenge der Milchpumpe 6 verändert. Auch kann die Luftmenge zusätzlich durch die ansaugende Milchpumpe variiert werden.

Hinter dem Wärmeüberträger 20 ist vorzugsweise noch ein Rückschlagventil 23 angeordnet. Es ist ferner vorteilhaft, wenn sämtliche Abschnitte von Kaltwasser spülbar sind, wozu entsprechende Zuleitungen und Ventile vorgesehen sein können (hier nicht dargestellt).

Mit einem ansteuerbaren Absperrventil 24 (Fig. 2) in der Leitung 10 kann die Zufuhr von Luft in der Leitung 10 unterbrochen werden. Dann können heiße, warme und kalte Milch, je nach Nutzen von Heißdampf, Wärmeüberträger 20 oder keiner Erwärmung, ausgegeben werden. Vorzugsweise werden in einen zunächst bereitgestellten Kaffee zwei verschieden temperierte Milchschäume gegeben oder es werden erst die zwei verschieden temperierten Milchschäume bereitgestellt und es wird dann ein Kaffee dazu gegeben.

**Bezugszeichenliste**

| | |
|---|---|
| Milchbehälter | 1 |
| Auslassvorrichtung | 2 |
| Milchleitung | 3 |
| Absperrventil | 4 |
| erste Mischkammer | 5 |
| Milchpumpe | 6 |
| Rückschlagventil | 7 |
| Zweite Mischkammer | 8 |
| Nachbehandlungsvorrichtung, Homogenisator | 9 |
| Luftleitung | 10 |
| Dampfzuleitung | 11 |
| Rückschlagventil | 12 |
| Zweigleitungen | 13, 13a, 13b, 14 |
| Luftquelle | 15, 15a, b |
| Luftquelle | 16 |
| einstellbare Drossel | 17 |
| Leitungsabschnitt | 18 |
| Leitungsabschnitt | 19 |
| Wärmeüberträger | 20 |
| Umschaltventil | 21 |
| Dampfquelle | 22 |
| Rückschlagventil | 23 |
| Absperrventil | 24 |
| Homogenisator | 95 |

## Patentansprüche

1. Verfahren, mit dem Milch-Luft-Emulsionen, insbesondere Milchschäume, mit einer Vorrichtung zum Erzeugen eines Milchschaums hergestellt werden, wobei die Vorrichtung zumindest aufweist:
a) eine von Milch durchflossenen Milchleitung (3),
b) wenigstens eine Einrichtung zur Erzeugung einer Milch-Luft-Emulsion als kalter Milchschaum - KMS - mit einer ersten Temperatur T1,
c) eine Dampfquelle (22) zur Injektion von Heißdampf in eine Milch-Luft-Emulsion erster Temperatur T1 auf eine heiße Temperatur T3 > T1 zum Erzeugen eines heißen Milchschaums - HMS - mit der Temperatur T3, so dass mittels Dampfinjektion der heiße Milchschaum - HMS - auf die Temperatur T3 erhitzbar ist, wobei T3 insbesondere zwischen 55° C und 80°C liegt, und
d) einen Wärmeüberträger (20) zum Erwärmen einer Milch-Luft-Emulsion erster Temperatur T1 auf eine warme Temperatur T2 zum Erzeugen eines warmen Milchschaums - WMS - mit der Temperatur T2, wobei gilt: T1 < T2 < T3; wobei mit dem Wärmeüberträger (20) der Milchschaum auf die Temperatur T2 auf eine denaturierend wirkende Temperatur, insbesondere auf zwischen 40° C und 54°C erwärmbar ist,
wobei das Verfahren zumindest folgende Schritte aufweist:
A) Fördern von Milch in der Milchleitung (3) mittels einer Milchpumpe (6), und gleichzeitig Ansaugen von Luft durch eine Luftleitung (10, 13, 14),
B) Mischen von Milch und Luft - vorzugsweise in einer ersten Mischkammer (5) - zum Erzeugen einer Luft-Milch-Emulsion als kalter Milchschaum - KMS - mit einer Temperatur T1 ,
C) Zusetzen von Dampf zur Luft-Milch-Emulsion zum Erhitzen der Luft-Milch-Emulsion auf eine heiße Temperatur T3, um einen heißen Milchschaum - HMS - mit der heißen Temperatur T3 > T1 zu erzeugen, wobei mittels Dampfinjektion der heiße Milchschaum - HMS - auf die heiße Temperatur T3 insbesondere zwischen 55° C und 80°C erhitzt wird;
D) wobei vor oder nach Schritt c) ein Erwärmen der Luft-Milch-Emulsion mit der Temperatur T1 mit einem Wärmeüberträger auf eine warme Temperatur T2 > T1 erfolgt, um ferner einen warmen Milchschaum - WMS - mit der warmen Temperatur T2 zu erzeugen, wobei gilt: T1 < T2 < T3, wobei mit dem Wärmeüberträger der warme Milchschaum - WMS - auf die warme Temperatur T2 insbesondere zwischen 40° C und 54°C erwärmt wird.

2. Verfahren nach Anspruch 1, **dadurch gekennzeichnet, dass** der kalte Milchschaum - KMS - mit einer Temperatur T1 < 30° C erzeugt wird.

3. Verfahren nach einem der vorstehenden Ansprüche, **dadurch gekennzeichnet, dass** als der Wärmeüberträger (20) ein vorzugsweise dampf- oder fluidbetriebener indirekter Wärmeüberträger verwendet wird, der, wenn aktiviert, den kalten Milchschaum - KMS - indirekt auf die Temperatur T2 erwärmt.

4. Verfahren nach einem der vorstehenden Ansprüche, **dadurch gekennzeichnet, dass** der Wärmeüberträger (20) als Mantelrohr-Wärmeüberträger (Fig. 2a), als Schicht-Wärmeüberträger (Fig. 2b) oder als Spiralrohr- Wärmeüberträger (Fig. 2c) ausgebildet ist und/oder der Wärmeüberträger (20) derart ausgelegt ist, dass in seinem Bereich der Volumenstrom des Milchschaums in der Einheit reduziert ist.

5. Verfahren nach einem der vorstehenden Ansprüche, **dadurch gekennzeichnet, dass** wenigstens eine Milchpumpe (6) zur Förderung der Milch verwendet wird.

6. Verfahren nach einem der vorstehenden Ansprüche, **dadurch gekennzeichnet, dass** die Einrichtung zur Erzeugung des kalten Milchschaums - KMS - mit der ersten kalten Temperatur T1 wenigstens eine erste Mischkammer (5) zum Mischen von Milch und Luft in der ersten Mischkammer (5) zum Erzeugen einer Luft-Milch-Emulsion als der kalte Milchschaum T1 mit der Temperatur T1 aufweist.

7. Verfahren nach einem der vorstehenden Ansprüche, **dadurch gekennzeichnet, dass** die Dampfquelle in einen ersten Leitungsabschnitt (18) der Milchleitung (3) mündet und dass der Wärmeüberträger in einem dazu parallel geschalteten Leitungsabschnitt (19) der Milchleitung (3) angeordnet ist.

8. Verfahren nach einem der vorstehenden Ansprüche 5 bis 7, **dadurch gekennzeichnet, dass** ein Umschaltventil (21) zum Umschalten zwischen den beiden Leitungsabschnitten vorgesehen ist und/oder dass die beiden parallel geschalteten Leitungsabschnitte (18, 19) hinter der Milchpumpe (6) angeordnet sind.

9. Verfahren nach einem der vorstehenden Ansprüche, **dadurch gekennzeichnet, dass** - vorzugsweise zwischen den beiden parallelen Leitungsabschnitten (18, 19) und einem Auslass (2) für die Milch-Luft-Emulsion - eine Nachbehandlungseinrichtung, insbesondere ein Homogenisator (95) zum Verfeinern der Konsistenz des Milchschaums vorgesehen ist.

10. Verfahren nach einem der vorstehenden Ansprüche, **dadurch gekennzeichnet, dass** sie eine oder mehrere Luftleitung (13a, 13b und/oder 14) zum Zuführen von Luft verschiedener Menge aufweist, wobei vorzugsweise in zumindest einer der Luftleitungen eine Drossel, insbesondere eine einstellbare Drossel angeordnet ist.

11. Verwendung eines Heißgetränkeautomaten zum Herstellen eines Kaffee-Milch-Mischgetränkes mit einer Kaffee-Zubereitungseinrichtung und mit einer Vorrichtung zum Erzeugen von Milchschaum, wobei die Vorrichtung zumindest aufweist:
a) eine von Milch durchflossenen Milchleitung (3),
b) wenigstens eine Einrichtung zur Erzeugung einer Milch-Luft-Emulsion als kalter Milchschaum - KMS - mit einer ersten Temperatur T1,
c) eine Dampfquelle (22) zur Injektion von Heißdampf in eine Milch-Luft-Emulsion erster Temperatur T1 auf eine heiße Temperatur T3 > T1 zum Erzeugen eines heißen Milchschaums - HMS - mit der Temperatur T3, so dass mittels Dampfinjektion der heiße Milchschaum - HMS - auf die Temperatur T3 zwischen 55° C und 80°C erhitzbar ist, und
d) einen Wärmeüberträger (20) zum Erwärmen einer Milch-Luft-Emulsion erster Temperatur T1 auf eine warme Temperatur T2 zum Erzeugen eines warmen Milchschaums - WMS - mit der Temperatur T2, wobei gilt: T1 < T2 < T3; wobei mit dem Wärmeüberträger (20) der Milchschaum auf die Temperatur T2 auf eine denaturierend wirkende Temperatur, insbesondere auf zwischen 40° C und 54°C erwärmbar ist,
wobei das Verfahren folgende Schritte aufweist: in einen Kaffee werden wenigstens ein warmer Milchschaum - WMS - und ein heißer Milchschaum - HMS - mit verschiedener Temperatur T2 und T3 ein- und zugegeben.

12. Verwendung nach Anspruch 11, **dadurch gekennzeichnet, dass** als der Wärmeüberträger (20) ein vorzugsweise dampf- oder fluidbetriebener indirekter Wärmeüberträger verwendet wird, der, wenn aktiviert, den kalten Milchschaum - KMS - indirekt auf die Temperatur T2 erwärmt, wobei der Wärmeüberträger (20) als Mantelrohr-Wärmeüberträger (Fig. 2a), als Schicht-Wärmeüberträger (Fig. 2b) oder als Spiralrohr- Wärmeüberträger (Fig. 2c) ausgebildet ist.

13. Verwendung nach einem der vorstehenden Ansprüche 11 oder 12, **dadurch gekennzeichnet, dass** der Wärmeüberträger (20) derart ausgelegt ist, dass in seinem Bereich der Volumenstrom des Milchschaums in der Einheit reduziert ist.

14. Verwendung nach einem der vorstehenden Ansprüche 11 bis 13, **dadurch gekennzeichnet, dass** wenigstens eine Milchpumpe (6) zur Förderung der Milch verwendet wird.

15. Verwendung nach einem der vorstehenden Ansprüche 11 bis 14, **dadurch gekennzeichnet, dass** die Einrichtung zur Erzeugung des kalten Milchschaums - KMS - mit der ersten kalten Temperatur T1 wenigstens eine erste Mischkammer (5) zum Mischen von Milch und Luft in der ersten Mischkammer (5) zum Erzeugen einer Luft-Milch-Emulsion als der kalte Milchschaum T1 mit der Temperatur T1 aufweist.

16. Verwendung nach einem der vorstehenden Ansprüche 11 bis 15, **dadurch gekennzeichnet, dass** die Dampfquelle in einen ersten Leitungsabschnitt (18) der Milchleitung (3) mündet und dass der Wärmeüberträger in einem dazu parallel geschalteten Leitungsabschnitt (19) der Milchleitung (3) angeordnet ist und/oder dass ein Umschaltventil (21) zum Umschalten zwischen den beiden Leitungsabschnitten vorgesehen ist.

17. Verwendung nach einem der vorstehenden Ansprüche 14 bis 16, **dadurch gekennzeichnet, dass** die beiden parallel geschalteten Leitungsabschnitte (18, 19) hinter der Milchpumpe (6) angeordnet sind, wobei - vorzugsweise zwischen den beiden parallelen Leitungsabschnitten (18, 19) und einem Auslass (2) für die Milch-Luft-Emulsion - eine Nachbehandlungseinrichtung, insbesondere ein Homogenisator (95) zum Verfeinern der Konsistenz des Milchschaums vorgesehen ist.

## Claims

1. Method by which milk/air emulsions, particularly milk foams, are produced by a device for producing a milk foam, wherein the device comprises at least:
a) a milk duct (3) flowed through by milk,
b) at least one device for producing a milk/air emulsion as a cold milk foam with a first temperature T1,
c) a steam source (22) for injection of hot steam into a milk/air emulsion of first temperature T1 at a hot temperature T3 > T1 for producing a hot milk foam with the temperature T3 so that the hot milk foam is heatable to the temperature T3 by means of steam injection, wherein T3 lies, in particular, between 55° C and 80° C, and
d) a heat exchanger (20) for warming a milk/air emulsion of first temperature T1 to a warm temperature T2 for producing a warm milk foam with the temperature T2, wherein T1 < T2 < T3, wherein the milk foam is can be warmed by the heat exchanger (20) to the temperature T2 to a temperature having a denaturation effect, particularly to between 40° C and 54° C,
wherein the method comprises at least the following steps:
A) conveying milk in the milk duct (3) by means of a milk pump (6) and at the same time inducting air through an air duct (10, 13, 14),
B) mixing milk and air - preferably in a first mixing chamber (5) - for producing an air/milk emulsion as cold milk foam with a temperature T1,
C) adding steam to the air/milk emulsion for heating the air/milk emulsion to a hot temperature T3 in order to produce a hot milk foam with the hot temperature T3 > T1, wherein the hot milk foam is heated by means of steam injection of the hot milk foam to the hot temperature T3, particularly between 55° C and 80° C; and
D) wherein prior to or after step c) warming the air/milk emulsion with the temperature T1 by a heat exchanger to a warm temperature T2 > T1 is carried out in order to further produce a warm milk foam with the warm temperature T2, wherein T1 < T2 < T3, wherein the warm milk foam is warmed by the heat exchanger to the warm temperature T2, particularly between 40° C and 54° C.

2. Method according to claim 1, **characterised in that** the cold milk foam is produced with a temperature T1 < 30° C.

3. Method according to one of the preceding claims, **characterised in that** an indirect heat exchanger which is preferably operated by steam or fluid and which when activated warms the cold milk foam indirectly to the temperature T2 is used as the heat exchanger (20).

4. Method according to any one of the preceding claims, **characterised in that** the heat exchanger (20) is constructed as a cased pipe heat exchanger (Fig. 2a), as a layer heat exchanger (Fig. 2b) or as a helical pipe heat exchanger (Fig. 2c) and/or the heat exchanger (20) is configured in such a way that in its region the volume flow of the milk foam in the unit is reduced.

5. Method according to any one of the preceding claims, **characterised in that** at least one milk pump (6) for conveying the milk is used.

6. Method according to any one of the preceding claims, **characterised in that** the device for producing the cold milk foam with the first old temperature T1 comprises at least one first milk chamber (5) for mixing milk and air in the first mixing chamber (5) for producing an air/milk emulsion as the cold milk foam T1 with the temperature T1.

7. Method according to any one of the preceding claims, **characterised in that** the steam source opens into a first duct section (18) of the milk duct (3) and that the heat exchanger is arranged in a duct section (19), which is connected in parallel therewith, of the milk duct (3).

8. Method according to any one of the preceding claims 5 to 7, **characterised in that** a changeover valve (21) for switching over between the two duct sections is provided and/or that the two duct sections (18, 19) connected in parallel are arranged behind the milk pump (6).

9. Method according to any one of the preceding claims, **characterised in that** a post-treatment device, particularly a homogeniser (95) for refining the consistency of the milk foam, is provided, preferably between the two parallel duct sections (18, 19) and an outlet (2) for the milk/air emulsion.

10. Method according to any one of the preceding claims, **characterised in that** it comprises one or more air ducts (13a, 13b and/or 14) for the supply of air of different quantities, wherein a throttle, particularly a settable throttle, is preferably arranged in at least one of the air ducts.

11. Use of a hot drinks machine for producing a coffee/milk mixed beverage with a coffee preparation device and with a device for producing milk foam, wherein the device at least comprises:
a) a milk duct (3) flowed through by milk,
b) at least one device for producing a milk/air emulsion as cold milk foam with a first temperature T1,
c) a steam source (22) for injection of hot steam into a milk/air emulsion of first temperature T1 at a hot temperature T3 > T1 for producing a hot milk foam with the temperature T3 so that the hot milk foam is heatable by means of steam injection to the temperature T3 between 55° C and 80° C, and
d) a heat exchanger (20) for warming a milk/air emulsion of first temperature T1 to a warm temperature T2 for producing a warm milk foam with the temperature T2, wherein T1 < T2 < T3, wherein the milk foam can be warmed by the heat exchanger (20) to the temperature T2 to a temperature having a denaturation effect, particularly to between 40° C and 54° C,
wherein the method comprises the following steps: at least one warm milk foam and hot milk foam with different temperatures T1 and T2 are introduced into and added to a coffee.

12. Use according to claim 11, **characterised in that** an indirect heat exchanger, which is preferably operated by steam or fluid, is used as the heat exchanger (20) and when activated warms the cold milk foam indirectly to the temperature T2, wherein the heat exchanger (20) is constructed as a cased pipe heat exchanger (Fig. 2a), as a layer heat exchanger (Fig. 2b) or as a helical pipe heat exchanger (Fig. 2c).

13. Use according to one of the preceding claims 11 and 12, **characterised in that** the heat exchanger (20) has such a configuration that in its region the volume flow of the milk foam is reduced in the unit.

14. Use according to any one of the preceding claims 11 to 13, **characterised in that** at least one milk pump (6) is used for conveying the milk.

15. Use according to any one of the preceding claims 11 to 14, **characterised in that** the device for producing the cold milk foam with the first cold temperature T1 has at least one first milk chamber (5) for mixing milk and air in the first mixing chamber (5) for producing an air/milk emulsion as the cold milk foam T1 with the temperature T1.

16. Use according to any one of the preceding claims 11 to 15, **characterised in that** the steam source opens into a first duct section (18) of the milk duct (3) and that the heat exchanger is arranged in a duct section (19), which is connected in parallel thereto, of the milk duct (3) and/or that a changeover valve (21) for switching over between the two duct sections is provided.

17. Use according to any one of the preceding claims 14 to 16, **characterised in that** the two duct sections (18, 19) connected in parallel are arranged behind the milk pump (6), wherein a post-treatment device, particularly a homogeniser (95) for refining the consistency of the milk foam, is provided preferably between the two parallel duct sections (18, 19) and an outlet (2) for the milk/air emulsion.

## Revendications

1. Procédé permettant d'obtenir des émulsions lait-air, en particulier des mousses de lait avec un dispositif d'obtention d'une mousse de lait, ce dispositif comprenant au moins :
(a) une conduite de lait (3), dans laquelle circule du lait,
(b) au moins un dispositif d'obtention d'une émulsion lait-air constituant une mousse de lait froide - KMS - ayant une première température T1,
(c) une source de vapeur (22) pour l'injection dans une émulsion lait-air ayant la première température T1 de vapeur chaude à une température chaude T3 > T1 pour l'obtention d'une mousse de lait chaude - HMS - à la température T3 de sorte que l'injection de vapeur permette de chauffer la mousse de lait chaude - HMS - à la température T3, la température T3 étant en particulier comprise entre 55°C et 80°C, et
(d) un échangeur de chaleur (20) permettant de réchauffer une émulsion lait-air ayant la première température T1 à une température réchauffée T2 pour obtenir une mousse de lait réchauffée - WMS - ayant la température T2, avec T1 < T2 < T3, l'échangeur de chaleur (20) permettant de réchauffer la mousse de lait à la température T2 ayant une action dénaturante, en particulier comprise entre 40°C et 54°C,
le procédé comprenant au moins les étapes suivantes consistant à :
A) refouler du lait dans la conduite de lait (3) au moyen d'une pompe à lait (6), et simultanément aspirer de l'air par une conduite d'air (10, 13, 14),
B) mélanger du lait et de l'air - de préférence dans une première chambre de mélange (5) - pour obtenir une émulsion air-lait constituant une mousse de lait froide -KMS- ayant une température T1,
C) ajouter de la vapeur à l'émulsion air-lait pour chauffer l'émulsion air-lait à une température chaude T3 pour obtenir une mousse de lait chaude - HMS - ayant la température chaude T3 > T1, l'injection de vapeur permettant de chauffer la mousse de lait chaude - HMS - à la température chaude T3 en particulier entre 55°C et 80°C,
D) avant ou après l'étape c), un réchauffage de l'émission air-lait ayant la température T1 avec un échangeur de chaleur à une température réchauffée T2 > T1 étant effectué pour obtenir ensuite une mousse de lait réchauffée - WMS - à la température réchauffée T2, avec T1< T2< T3, l'échangeur de chaleur permettant de réchauffer la mousse de lait réchauffée - WMS - à la température réchauffée T2 en particulier entre 40°C et 54°C.

2. Procédé conforme à la revendication 1,
**caractérisé en ce que**
la mousse de lait froide - KMS - est obtenue à une température T1 < 30°C.

3. Procédé conforme à l'une des revendications précédentes, **caractérisé en ce que**
en tant qu'échangeur de chaleur (20) on utilise un échangeur de chaleur indirect de préférence actionné par de la vapeur ou un fluide, qui, lorsqu'il est activé réchauffe indirectement la mousse de lait froide - KMS - à la température T2.

4. Procédé conforme à l'une des revendications précédentes,
**caractérisé en ce que**
l'échangeur de chaleur (20) est réalisé sous la forme d'un échangeur de chaleur à tube enveloppe (Fig. 2a), sous la forme d'un échangeur de chaleur à couches (Fig. 2b) ou sous la forme d'un échangeur de chaleur à tube hélicoïdal (Fig. 2c), et/ou l'échangeur de chaleur (20) est réalisé de sorte que, dans sa zone le débit de la mousse de lait dans l'unité soit réduite.

5. Procédé conforme à l'une des revendications précédentes,
**caractérisé en ce que**
l'on utilise au moins une pompe à lait (6) pour le refoulement du lait.

6. Procédé conforme à l'une des revendications précédentes,
**caractérisé en ce que**
le dispositif d'obtention de la mousse de lait froide - KMS - ayant la première température froide T1 comporte au moins une première chambre de mélange (5) pour mélanger du lait et de l'air dans celle-ci de façon à obtenir une émulsion air-lait constituant la mousse de lait froide ayant la température T1.

7. Procédé conforme à l'une des revendications précédentes,
**caractérisé en ce que**
la source de vapeur débouche dans un premier segment (18) de la conduite de lait (3) et l'échangeur de chaleur est monté dans un segment (19) branché en parallèle de celui-ci de la conduite de lait (3).

8. Procédé conforme à l'une des revendications précédentes 5 à 7,
**caractérisé en ce qu'**
il est prévu une soupape d'inversion (21) pour permettre d'effectuer une commutation entre les deux segments de conduite, et/ou les deux segments de conduite (18, 19) branchés en parallèle sont montés à l'arrière de la pompe à lait (6).

9. Procédé conforme à l'une des revendications précédentes,
**caractérisé en ce qu'**
il est prévu - de préférence entre les deux segments de conduite (18, 19) branchés en parallèle et une sortie (2) de l'émulsion lait-air - un dispositif de post traitement, en particulier un homogénéisateur (95) pour permettre d'affiner la consistance de la mousse de lait.

10. Procédé conforme à l'une des revendications précédentes,
**caractérisé en ce qu'**
il est prévu une ou plusieurs conduite(s) d'air (13a, 13b et/ou 14) pour permettre de fournir différentes quantités d'air, et de préférence, dans au moins l'une des conduites d'air est monté un étranglement, en particulier un étranglement réglable.

11. Utilisation d'un appareil automatique de préparation de boissons chaudes pour obtenir une boisson mixte café lait avec un appareil de préparation de café et un dispositif obtention d'une mousse de lait, ce dispositif comprenant au moins :
a) une conduite de lait (3) dans laquelle circule du lait,
b) au moins un dispositif d'obtention d'une émulsion lait-air constituant une mousse de lait froide - KMS - ayant une première température T1,
c) une source de vapeur (22) l'injection dans une émulsion lait-air ayant la première température T1 de vapeur chaude à une température chaude T3 > T1 pour obtenir une mousse de lait chaude - HMS - ayant la température T3 de sorte que, l'injection de vapeur permette de chauffer la mousse de lait chaude - HMS - à une température T3 comprise entre 55°C et 80°C, et
d) un échangeur de chaleur (20) permettant de réchauffer une émulsion lait-air ayant la première température T1 à une température réchauffée T2 pour obtenir une mousse de lait réchauffée - WMS - ayant la température T2 avec T1 < T2 < T3, l'échangeur de chaleur (20) permettant de réchauffer la mousse de lait à la température T2 ayant une action dénaturante, en particulier comprise entre 40°C et 54°C,
le procédé comprenant des étapes consistant à :
incorporer et à ajouter dans un café au moins une mousse de lait réchauffée - WMS - et une mousse de lait chaude - HMS - ayant des températures différentes T2 et T3.

12. Utilisation conforme à la revendication 11,
**caractérisé en ce qu'**
en tant qu'échangeur de chaleur (20) on utilise un échangeur de chaleur indirect de préférence actionné par de la vapeur ou par un fluide qui, lorsqu'il est activé permet de réchauffer la mousse de lait froide - KMS - indirectement à la température T2, l'échangeur de chaleur (20) étant réalisé sous la forme d'un échangeur de chaleur à tube enveloppe (Fig. 2a), sous la forme d'un échangeur de chaleur à couches (Fig. 2b), ou sous la forme d'un échangeur de chaleur à tube hélicoïdal (Fig. 2c).

13. Utilisation conforme à l'une des revendications précédentes 11 et 12,
**caractérisée en ce que**
l'échangeur de chaleur (20) est réalisé de sorte que dans sa zone le débit de la mousse de lait dans l'unité soit réduit.

14. Utilisation conforme à l'une des revendications précédentes 11 à 13,
**caractérisée en ce que**
l'on utilise au moins une pompe à lait (6) pour le refoulement du lait.

15. Utilisation conforme à l'une des revendications précédentes 11 à 14,
**caractérisée en ce que**
le dispositif d'obtention de la mousse de lait froide - KMS - ayant la première température froide T1 comporte au moins une première chambre de mélange (5) pour mélanger le lait et l'air dans celle-ci de façon à obtenir une émulsion air-lait constituant la mousse de lait froide T1 ayant la température T1.

16. Utilisation, conforme à l'une des revendications précédentes 11 à 15,
**caractérisée en ce que**
la source de vapeur débouche dans un premier segment (18) de la conduite de lait (3) et l'échangeur de chaleur est monté dans un segment (19) de la conduite de lait (3) branché en parallèle à celui-ci, et/ou il est prévu une soupape d'inversion (21) pour permettre d'effectuer une commutation entre les deux segments de conduite.

17. Utilisation conforme à l'une des revendications précédentes 14 à 16,
**caractérisée en ce que**
les deux segments de conduite branchés en parallèle (18, 19) sont montés à l'arrière de la pompe à lait (6), et, il est prévu, de préférence entre les deux segments de conduite (18, 19) branchés en parallèle et une sortie (2) de l'émulsion lait-air, un dispositif de poste-traitement en particulier un homogénéisateur (95) permettant d'affiner la consistance de la mousse de lait.
